# FASCICULE DE BREVET EUROPEEN

(11) **EP 2 655 454 B1**
(45) Date de publication et mention de la délivrance du brevet: **01.06.2016**
(21) Numéro de dépôt: 11815516.7
(22) Date de dépôt: 22.12.2011
(51) Int. Cl.: C08F 293/00, C08F 2/38, C08F 2/22, C09K 8/035, D01D 5/38, A61K 8/90, C09D 153/00, C09K 8/588, D01F 6/36, D01F 6/42, E21B 43/16

(54) **PARTICULES POLYMERIQUES FILEMENTEUSES ET LEUR UTILISATION COMME MODIFICATEURS DE RHEOLOGIE**
FILAMENTÖSE POLYMERTEILCHEN UND IHRE VERWENDUNG ALS RHEOLOGIEMODIFIKATOREN
FILAMENTOUS POLYMER PARTICLES AND USE THEREOF AS RHEOLOGY MODIFIERS

(30) Priorité: 23.12.2010 FR 1061191
(43) Date de publication de la demande: 30.10.2013
(73) Titulaire: Arkema France, 92700 Colombes (FR); Centre National de la Recherche Scientifique, 75016 Paris (FR); Université Pierre et Marie Curie (Paris 6), 75005 Paris (FR); Université Claude Bernard Lyon 1, 69100 Villeurbanne (FR); Ecole Superieure de Chimie-Physique-Electronic de Lyon, 69616 Villeurbanne, Cedex (FR)
(72) Inventeur: MAGNET, Stéphanie, F-64370 Morlanne (FR); INOUBLI, Raber, F-64000 Pau (FR); COUVREUR, Laurence, F-75009 Paris (FR); CHARLEUX, Bernadette, F-69003 Lyon (FR); BRUSSEAU, Ségolène, F-94230 Cachan (FR)
(74) Mandataire: Albani, Dalila
(86) Numéro de dépôt international: PCT/FR2011/053145
(87) Numéro de publication internationale: WO 2012/085473

(56) Documents cités:
- DE-A1- 3 418 397
- FR-A1- 2 863 617
- FR-A1- 2 931 153
- US-A- 4 584 358

## Description

La présente invention concerne des particules polymériques sous forme de filaments constitués de copolymères à blocs, ainsi que leur utilisation comme modificateurs de rhéologie de solutions aqueuses ou organiques.

Lorsqu'un gisement pétrolier ou de gaz naturel atteint la fin de sa durée de vie normale, la majeure partie des hydrocarbures (jusqu'aux deux tiers) reste dans le sol parce qu'ils sont trop difficiles ou trop chers à extraire. Il est estimé qu'en en récupérant seulement 1 % de plus à travers le monde, ceci équivaudrait à 20-30 milliards de barils de pétrole supplémentaires.

Les techniques de récupération assistée des hydrocarbures (pétrole et gaz naturel) par voie chimique consistent à injecter une phase aqueuse dans le réservoir souterrain par des puits injecteurs, situés à distance des puits producteurs. La phase aqueuse injectée permet de maintenir la pression dans le réservoir et de déplacer les hydrocarbures vers les puits de production. On vise aussi à augmenter la fluidité du pétrole qu'on cherche à récupérer ou à diminuer la perméabilité de certaines couches du sous-sol dont les caractéristiques nuisent à un balayage efficace du réservoir.

L'efficacité de ces méthodes se trouve limitée par la différence de viscosité entre le pétrole et l'eau, qui amène l'eau à chercher à passer directement du puits d'injection au puits de production.

La phase aqueuse destinée à être injectée dans un puits est le plus souvent additionnée de produits chimiques dont le rôle est d'augmenter sa viscosité, ce qui permet de mieux balayer l'ensemble du gisement. L'augmentation de la viscosité se traduit par une réduction du rapport de mobilité entre la phase aqueuse et la phase hydrocarbonée.

Il est connu par l'homme de l'art qu'une augmentation de la viscosité d'une solution aqueuse avec de très faibles taux d'additif peut être obtenue par l'utilisation de polymères hydrosolubles de très grande masse molaire et/ou possédant des unités monomérique chargées (notamment par des groupement acide) ou par l'utilisation de biopolymères hydrophiles donnant des structures rigides.

Les polymères hydrosolubles chargés (comme les HPMA ou «high molecular weight polyacrylamide » qui sont des acrylamides copolymérisés avec un monomère ionique) présentent un caractère viscosifiant par l'augmentation importante du rayon de giration de la molécule engendré par les interactions répulsives des charges présentes dans la molécule. La présence de sels ou une variation de pH du milieu peut «écranter » ces charges, supprimer ces interactions et ainsi supprimer l'effet viscosifiant.

Les biopolymères hydrophiles tels que le scléroglucanne sont des modificateurs de rhéologie très efficaces, mais présentent une sensibilité très importante à la dégradation bactérienne. Ces molécules sont « coupées » par certains micro-organismes et perdent ainsi toutes propriétés viscosifiantes et rhéo-fluidifiantes.

D'autres composés polymériques ont été utilisés comme modificateurs de rhéologie, par exemple les polymères hydrophobiquement associatifs (PHA) qui ont un squelette hydrophile et comportent le long des chaînes de faibles quantités de monomères hydrophobes susceptibles de s'associer dans l'eau sous la forme de nanodomaines hydrophobes. Ceux-ci agissent comme des points de réticulation temporaires et confèrent aux PHA un caractère rhéo-fluidifiant marqué.

La présente invention se propose de fournir de nouveaux composés polymériques aptes à modifier la rhéologie des solutions aqueuses ou organiques et qui pallient les inconvénients présentés ci-dessus.

Un premier but de la présente invention est de fournir des structures polymériques aptes, à un très faible taux, à modifier la rhéologie de l'eau d'injection dans le puits lors de la récupération tertiaire des hydrocarbures. Un autre but de l'invention est de fournir des structures polymériques filamenteuses aptes à garder leur morphologie après une dilution importante dans l'eau et/ou dans un solvant organique. Il a maintenant été trouvé que des particules polymériques sous forme de filaments constitués de copolymères à blocs présentent un caractère viscosifiant et rhéofluidifiant en milieu dispersé et ce, à une concentration très faible; de plus, l'effet viscosifiant et rhéofluidifiant des particules filamenteuses selon l'invention n'est pas affecté par la présence de sel ou par les variations de pH du milieu et lesdites particules ne sont pas sensibles à la dégradation bactérienne.

L'invention a pour objet, selon un premier aspect, des particules polymériques filamenteuses constituées de copolymères à blocs synthétisés par polymérisation radicalaire contrôlée en émulsion. De manière caractéristique, ces particules polymériques se présentent sous forme de cylindres ayant un ratio longueur/diamètre supérieur à 100.

La synthèse desdites particules est effectuée à partir d'au moins un monomère hydrophobe en présence d'un macroamorceur vivant dérivé d'un nitroxyde, caractérisé en ce que :
- lesdites particules filamenteuses sont obtenues en milieu aqueux en une seule étape directement lors de la synthèse desdits copolymères à blocs effectuée en chauffant le milieu de réaction à une température de 60 à 120°C,
- ledit macroamorceur est hydrosoluble,
- le pourcentage de la masse molaire du macroamorceur hydrosoluble dans le copolymère à blocs final est compris entre 10 et 50%, et en ce que
- le taux de conversion du monomère hydrophobe est d'au moins 50%.

Cette méthode directe de préparation de particules filamenteuses ne nécessite pas l'emploi de co-solvant organique.

Dans le cadre de la présente invention, le terme « particules filamenteuses » correspond à des assemblages de macromolécules amphiphiles qui, lorsqu'elles sont en suspension dans l'eau (autrement dit, lorsqu'elles forment une dispersion aqueuse), prennent la forme de filaments (autrement dit, de cylindres pleins et flexibles) dont le coeur est constitué des éléments hydrophobes et la surface des éléments hydrophiles desdites macromolécules. Ces particules filamenteuses sont observables au microscope électronique en transmission (TEM). Les images de microscopie montrent des filaments dont le diamètre est supérieur ou égal à 5 nm et la longueur est supérieure à 500 nm, de préférence supérieure à 1 micron, avantageusement supérieure à 5 microns. Selon un mode de réalisation, la longueur des particules filamenteuses selon l'invention est d'au moins 10 micromètres.

A la différence des particules filamenteuses, les particules (ou micelles) sphériques en suspension dans l'eau sont des assemblages de molécules amphiphiles qui prennent la forme d'une sphère pleine dont le coeur est constitué des éléments hydrophobes des molécules et la surface des éléments hydrophiles.

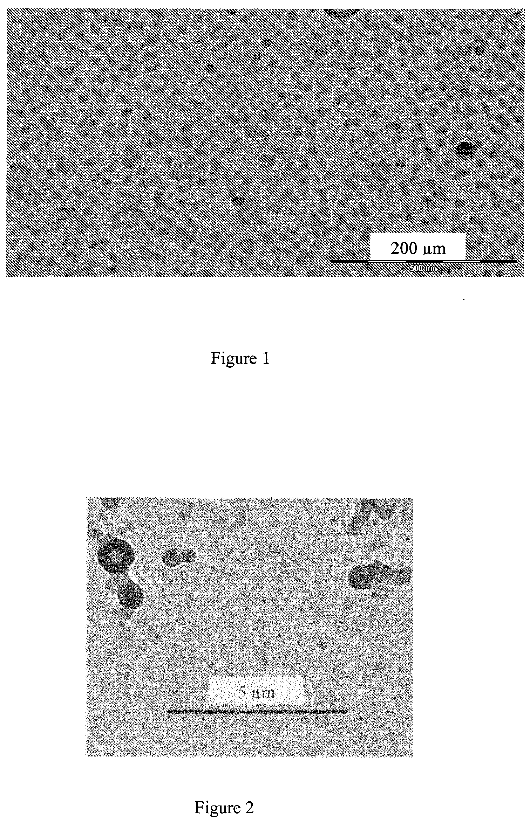

Dans le document WO 2009150367 la demanderesse avait déjà décrit la préparation de particules polymériques à partir d'au moins un monomère hydrophobe méthacrylate et d'un autre monomère hydrophobe en présence d'un macroamorceur vivant de type nitroxyde, dérivé du SG1. Les particules polymériques ainsi obtenues se présentent sous forme de micelles sphériques, quel que soit le taux de conversion. Elles sont caractérisées dans l'exemple 2 comparatif de la présente invention ainsi que dans la figure 1 annexée.

De manière surprenante il a maintenant été trouvé qu'en présence d'un macroamorceur hydrosoluble et en sélectionnant un ratio spécifique entre les masses molaires du macroamorceur hydrosoluble et du second bloc hydrophobe, des particules filamenteuses sont obtenues.

Avantageusement, les particules filamenteuses selon l'invention sont hors de l'équilibre thermodynamique (on dit qu'elles sont dans un état gelé) contrairement aux micelles vermiformes décrites précédemment qui, sous l'effet de la dilution, passent à l'état de micelles sphériques, qui est un état thermodynamique plus stable. Cet état hors équilibre thermodynamique est indépendant de la valeur de la transition vitreuse du polymère, mais dépendant en partie de la longueur du bloc hydrophobe. Les particules filamenteuses selon l'invention voient leur forme et structure se maintenir en milieu dispersé, indépendamment de leur concentration dans le milieu, de la dialyse des particules (figure 7), de la centrifugation du milieu (figure 8), des variations de pH (figure 9) ou de salinité de celui-ci (figure 10).

Selon un deuxième aspect, l'invention concerne un procédé pour modifier la rhéologie d'une solution aqueuse ou organique au moyen desdites particules polymériques filamenteuses. Ce procédé comprend une étape d'ajout de ces particules filamenteuses à une solution aqueuse ou organique à un taux massique allant de 100 à 10000 ppm.

Selon un autre aspect, l'invention a trait à un procédé d'extraction renforcée d'hydrocarbures au moyen d'un additif polymérique dans lequel on mélange ledit additif avec de l'eau ou de la saumure dans une proportion d'au moins 500 ppm d'additif puis on injecte ce mélange sous pression dans la roche ; de manière caractéristique, ledit additif consiste en les particules polymériques filamenteuses selon l'invention.

Selon encore un autre aspect, l'invention se rapporte à une composition pour modifier la rhéologie d'une solution aqueuse ou organique, ladite composition comprenant les particules polymériques filamenteuses décrites ci-dessus.

L'invention et les avantages qu'elle procure seront mieux compris à la lumière de la description détaillée qui va suivre et des figures annexées dans lesquelles :
- la figure 1 est l'image obtenue par TEM pour les particules sphériques de l'exemple comparatif 2, correspondant à l'invention décrite dans le document WO 2009150367 ; le taux de conversion massique est de 67,2%, et le taux massique de la partie hydrophile composant le copolymère à bloc (ou « taux de fraction hydrophile ») est de 23,4% en poids;
- la figure 2 correspond à l'image TEM de particules filamenteuses selon l'invention, pour un taux de conversion massique de 25,5% et un taux de fraction hydrophile de 31% en poids ;

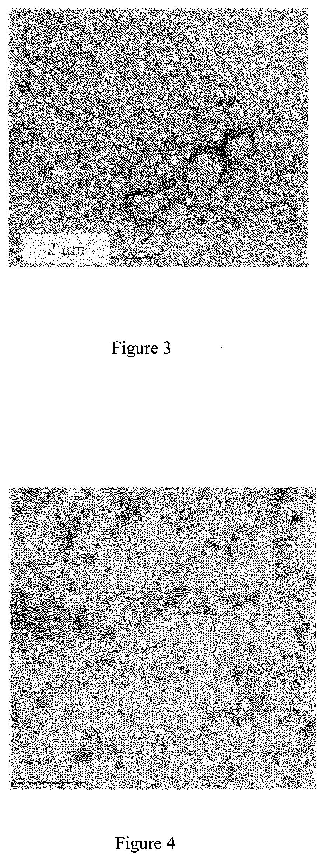

- la figure 3 correspond à l'image TEM de particules filamenteuses selon l'invention, pour un taux de conversion massique de 52% et un taux de fraction hydrophile de 18% en poids ;
- la figure 4 correspond à l'image TEM de particules filamenteuses selon l'invention, pour un taux de conversion massique de 68% et un taux de fraction hydrophile de 14,4% en poids ;

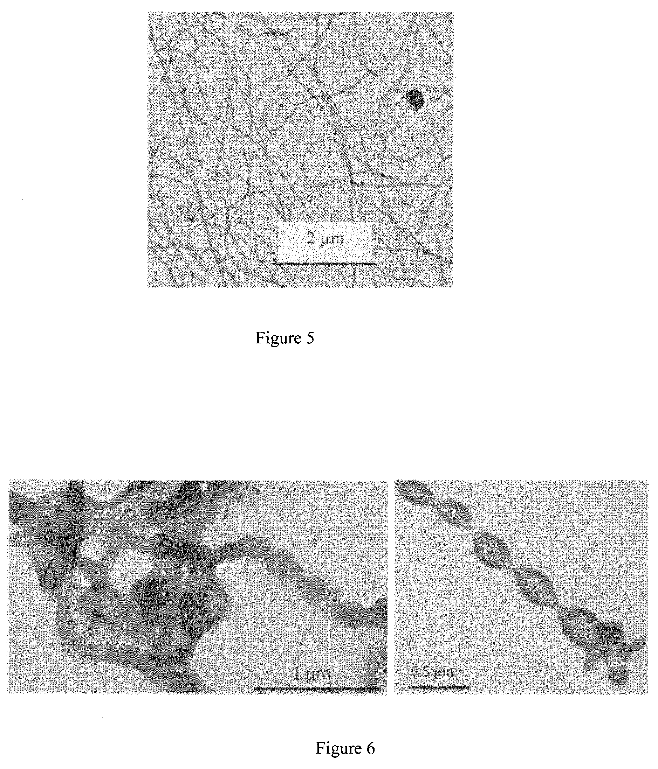

- la figure 5 illustre les particules filamenteuses réticulées selon l'invention, pour un taux de conversion massique de 68% et un taux de fraction hydrophile de 14,4% en poids, telles qu'observées au TEM ;
- la figure 6 correspond aux images TEM de particules filamenteuses sous forme de « poche » obtenues avec un copolymère poly(méthacrylate de sodium-*co*-styrène sulfonate de sodium)-*b*-poly(méthacrylate de méthyle-*co-*styrène) ;

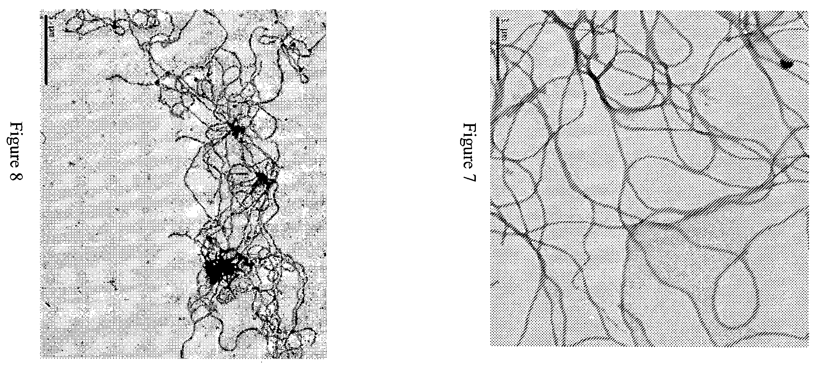

- la figure 7 correspond à l'image TEM de particules filamenteuses préparées dans une solution à 0,2% en poids dialysées pendant 4 jours contre de l'eau ultra pure ;
- la figure 8 correspond à l'image TEM de ces particules filamenteuses après centrifugation ;

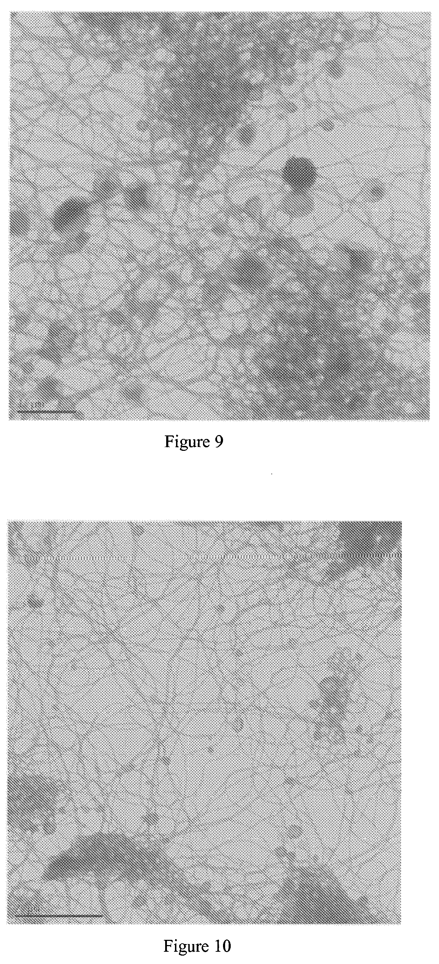

- la figure 9 correspond à l'image TEM de particules filamenteuses diluées dans une solution à pH 5 ;
- la figure 10 correspond à l'image TEM de particules filamenteuses diluées dans une solution aqueuse contenant 35 g de sel (NaCl) pour 1kg d'eau.

L'objet de la présente invention concerne les propriétés rhéologiques (caractère viscosifiant et rhéofluidifiant) en milieu dispersé de particules de copolymères ayant une forme bien spécifique de fibrille allongée. Il a maintenant été trouvé que de manière surprenante, certaines particules polymériques ont la propriété d'augmenter la viscosité de solutions aqueuses (ou organiques) et par la même occasion de leur donner un caractère rhéofluidifiant.

Le caractère viscosifiant à de très faible concentration est apporté par une pseudo-percolation de la structure en milieu dispersé obtenue à des très faibles concentrations. Le caractère rhéofluidiant est obtenu par une pseudo-désenchevetrement obtenu très rapidement (en fonction du gradient de déformation ou de cisaillement) grâce la rigidité et au très grand rapport entre la longueur et le rayon de la structure. De plus, grâce à son caractère gelé, cette structure de copolymère n'est pas sensible à la salinité ou à des variations de pH du milieu aqueux ou organique à viscosifier.

Par « rhéofluidification » on entend la diminution des propriétés rhéologiques (viscosité) sous l'effet d'une augmentation de la contrainte, du cisaillement ou de la déformation appliqués au système étudié.

A cet effet, l'invention a pour objet, selon un premier aspect, des particules polymériques filamenteuses ayant un ratio longueur/diamètre supérieur à 100, lesdites particules étant constituées de copolymères à blocs synthétisés par polymérisation radicalaire contrôlée en émulsion effectuée à partir d'au moins un monomère hydrophobe en présence d'un macroamorceur hydrosoluble.

La synthèse desdites particules étant effectuée à partir d'au moins un monomère hydrophobe en présence d'un macroamorceur vivant dérivé d'un nitroxyde.

De manière caractéristique, lesdites particules filamenteuses sont obtenues en milieu aqueux de synthèse desdits copolymères à blocs effectuée en chauffant le milieu de réaction à une température de 60 à 120°C, avec un pourcentage de la masse molaire du macroamorceur hydrophile dans le copolymère à blocs final compris entre 10 et 50%, le taux de conversion du monomère hydrophobe étant d'au moins 50%. Le pH initial du milieu aqueux peut varier entre 5 et 10. Cette méthode directe de préparation de particules filamenteuses ne nécessite pas l'emploi de co-solvant organique.

Par « macroamorceur vivant » on entend un polymère comprenant au moins une extrémité apte à être réengagée dans une réaction de polymérisation par ajout de monomères à une température et pression appropriées. Avantageusement, ledit macroamorceur est préparé par PRC. Par « macroamorceur hydrosoluble » on entend un polymère soluble dans l'eau comportant à son extrémité une fonction réactive capable de réamorcer une polymérisation radicalaire. Ce macroamorceur est composé majoritairement de monomères hydrophiles, c'est à dire des monomères présentant une ou plusieurs fonctions aptes à établir des liaisons hydrogène avec l'eau. Dans le cas de la polymérisation d'un monomère hydrophobe, on formera un copolymère amphiphile dont le bloc hydrophile sera constitué par le macroamorceur alors que le bloc hydrophobe sera issu de la polymérisation du (des) monomère(s) hydrophobes. Selon une variante de réalisation, ledit macroamorceur hydrosoluble préformé est ajouté au milieu de réaction comprenant au moins un monomère hydrophobe.

Selon une variante, ledit macroamorceur hydrosoluble est synthétisé dans le milieu de réaction aqueux en une étape préliminaire, sans isolement du macroamorceur formé ni élimination des éventuels monomères hydrophiles résiduels Cette deuxième variante est une polymérisation « one-pot ».

Les monomères hydrophobes peuvent être choisis parmi :
- les monomères vinylaromatiques tels que le styrène ou les styrènes substitués,
- les acrylates d'alkyle, de cycloalkyle ou d'aryle tels que l'acrylate de méthyle, d'éthyle, de butyle, d'éthyl-2 hexyle ou de phényle,
- les méthacrylates d'alkyle, de cycloalkyle, d'alkényle ou d'aryle tels que le méthacrylate de méthyle, de butyle, de lauryle, de cyclohexyle, d'allyle, d'éthyl-2 hexyle ou de phényle
- et la vinylpyridine.

Ces monomères hydrophobes sont ajoutés au milieu de réaction, qui comprend majoritairement de l'eau.

De manière préférée, le pourcentage de la masse molaire du macroamorceur hydrosoluble dans le copolymère à blocs final est compris entre 10 et 30%.

La mise en oeuvre du procédé selon l'invention permet d'obtenir des particules polymériques filamenteuses dans lesquelles le taux massique de la partie hydrophile composant le copolymère à bloc est inférieur à 25%.

Selon un mode de réalisation, lorsque le milieu de réaction est additionné d'un agent réticulant, on obtient des particules filamenteuses réticulées. Ledit agent réticulant est un comonomère réticulant différent des monomères hydrophobes précités.

Par comonomère réticulant, on entend un monomère qui, de par sa réactivité avec les autres monomères présents dans le milieu de polymérisation, est capable de générer un réseau tridimensionnel covalent. D'un point de vue chimique, un comonomère réticulant comprend généralement au moins deux fonctions polymérisables éthyléniques, qui en réagissant, sont susceptibles de créer des ponts entre plusieurs chaînes polymériques.

Ces comonomères réticulants peuvent être susceptibles de réagir avec les monomères hydrophobes insaturés lors de la synthèse desdites particules.

Parmi les comonomères réticulants, on peut citer les divinylbenzènes, les trivinylbenzènes, les (méth)acrylates allyliques, les (méth)acrylates de diallylmaléatepolyol tels que les tri(méth)acrylates de triméthylolpropane, les di(méth)acrylates d'alkylèneglycol qui ont de 2 à 10 atomes de carbone dans la chaîne carbonée tels que les di(méth)acrylates d'éthylèneglycol, les di(méth)acrylates de 1,4-butanediol, les di(méth)acrylates de 1,6-hexanediol, les N,N'-alkylène bisacrylamides, tels que le N,N'-méthylène bisacrylamide. De préférence, on utilisera comme agent réticulant le divinylbenzène ou un diméthacrylate.

De manière caractéristique, les particules filamenteuses selon l'invention présentent un pourcentage de la masse molaire du macroamorceur hydrophile dans le copolymère à blocs final compris entre 10 et 50%. Telles qu'observées par TEM, elles se présentent sous forme de fibres cylindriques ayant un ratio longueur/diamètre supérieur à 100 ; leur diamètre est constant sur toute leur longueur et est supérieur ou égal à 5 nm, tandis que leur longueur est supérieure à 500 nm, de préférence supérieure à 1 micron, avantageusement supérieure à 5 microns et de manière encore plus préférée elle est supérieure ou égale à 10 micromètres. Les particules filamenteuses selon l'invention voient leurs forme et structure se maintenir en milieu dispersé, indépendamment de leur concentration dans le milieu et/ou des variations de pH ou de salinité de celui-ci.

Selon un deuxième aspect, l'invention concerne un procédé pour modifier la rhéologie d'une solution aqueuse ou organique au moyen desdites particules polymériques filamenteuses. Ce procédé comprend une étape d'ajout de ces particules filamenteuses à une solution aqueuse ou organique à un taux massique de minimum 100 ppm, de préférence allant de 500 à 10000 ppm.

Selon un autre aspect, l'invention a trait à un procédé d'extraction renforcée d'hydrocarbures au moyen d'un additif polymérique dans lequel on mélange ledit additif avec de l'eau ou de la saumure dans une proportion d'au moins 500 ppm d'additif puis on injecte ce mélange sous pression dans la roche ; de manière caractéristique, ledit additif consiste en les particules polymériques filamenteuses selon l'invention.

Selon encore un autre aspect, l'invention se rapporte à une composition pour modifier la rhéologie d'une solution aqueuse ou organique destinée notamment à des applications dans la cosmétique ou les peintures ladite composition comprenant les particules polymériques filamenteuses décrites ci-dessus.

L'invention va maintenant être décrite à l'aide des exemples suivants donnés à titre illustratif et non limitatif.

### Exemple 1 : Préparation du macroamorceur poly(acide méthacrylique-co-styrène sulfonate de

### sodium)

L'exemple 1 illustre la préparation d'un copolymère vivant poly(acide méthacrylique-co-styrène sulfonate de sodium), utilisé comme macroamorceur, agent de contrôle et stabilisant pour la mise en oeuvre du procédé de l'invention.
Pour ce faire, un mélange contenant 75,2 g d'acide méthacrylique (2,0 mol.L⁻¹), 17,32 g de styrène sulfonate de sodium (0,18 mol.L⁻¹ soit f_{0,SS} = 0,087 et 398 g de DMSO est dégazé à température ambiante par un bullage d'azote. 3,782 g (2,27×10-2 mol.L⁻¹) de l'alcoxyamine BlocBuilder^{®}-MA (*N*-(2-methylpropyl)-*N*-(1-diethylphosphono-2,2-dimethylpropyl)-*O*-(2-carboxylprop-2-yl) hydroxylamine) est ensuite ajouté.

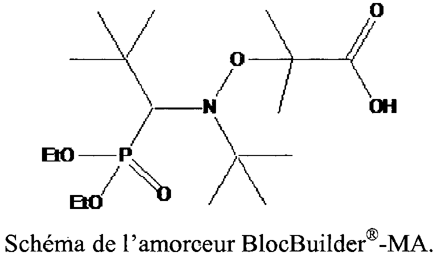

Le dégazage est poursuivi pendant 10 minutes. Le mélange dégazé est introduit dans un tricol de 1L, préchauffé à 75°C, surmonté d'un réfrigérant muni d'un bulleur, d'une entrée d'azote et d'un thermomètre. La polymérisation est réalisée à 76°C et le temps t=0 est déclenché lorsque la température atteint 35°C dans le milieu réactionnel. Le macroamorceur obtenu est le P(MAA-*co*-SS)-SG₁. La manipulation est stoppée après 16 minutes de réaction en plongeant le milieu sous agitation dans un erlenmeyer refroidit avec un bain de glace. Le milieu de réaction est ensuite précipité gouttes à gouttes, en deux fois, dans un volume total de 4,5 litres de dichlorométhane refroidit soumis à une vive agitation. Un précipité blanc apparaît dans le milieu. Le milieu est filtré sous fritté de porosité n°4 puis séché pendant 3 jours sous vide.

Des prélèvements sont réalisés au temps initial et final afin de :
- déterminer la cinétique de polymérisation (détermination de la conversion molaire et massique par RMN ¹H (DMSO d₆, 300 MHz) ;
- suivre l'évolution des masses molaires moyennes en nombre (Mn) en fonction de la conversion en monomères.

Le tableau 1 ci-dessous présente les caractéristiques du macroamorceur purifié

**Tableau 1**

| Temps (min) | Conversion (%) | Mₙ, ^{a)} expérimental (g.mol⁻¹) | M_{n,}^{b)} théorique (g.mol⁻¹) | Iₚ | Mn,^{c)} expérimental (g.mol⁻¹) |
|---|---|---|---|---|---|
| 16 | 10 | 7200 | 1 300 | 1,5 | 6350 |

(a) Déterminée par chromatographie d'exclusion stérique dans le DMF avec 1g.L⁻¹ de LiBr, avec une calibration au polyméthacrylate de méthyle, après purification et méthylation des unités acide méthacryliques en unités méthacrylate de méthyle ;
(b) Calculée à partir d'unités méthacrylate de méthyle ;
(c) Calculée à partir d'unités acide méthacrylique.

La Mₙ expérimentale est déterminée par chromatographie d'exclusion stérique dans le DMF contenant 1g/L de LiBr, avec calibration au polyméthacrylate de méthyle, après méthylation des unités acide méthacryliques en unités méthacrylate de méthyle. Le débit est à 0,8 mL/min avec le toluène comme marqueur de débit. Les échantillons sont préparés à une concentration de 5 mg/mL, sont filtrés sur des filtres de 0,45 µm et sont analysés sur des colonnes Polymer Standards Service Columns (GRAM de 30 - 1 000 Å).

L'indice de polydispersité Iₚ est calculé à partir d'unités méthacrylates de méthyle.

Le caractère vivant de ce macroamorceur est testé dans un premier temps par une analyse de RMN ³¹P. Cette technique de caractérisation montre la présence du phosphore dans le polymère purifié.

Une seconde expérience d'extension de chaîne est effectuée pour obtenir un copolymère à bloc de type poly(acide méthacrylique-*co-*styrène sulfonate de sodium)-*b*-poly(styrène).

Un mélange contenant 61,4 mg du macroamorceur précédent P(MAA-*co*-SS)-SG1 (3,08×10⁻³ mol.L⁻¹), 1,314 g de styrène (3,22 mol.L⁻¹), 2,7335 g de DMSO est dégazé à température ambiante par un bullage d'azote pendant 20 minutes dans un ballon de 10 mL. Le ballon est plongé dans un bain d'huile à 120°C pendant 5,5 heures.

Le polymère obtenu est analysé pour :
- déterminer la conversion de la polymérisation (détermination par gravimétrie) ;
- suivre la valeur de la masse molaire moyenne en nombre (Mn) en fonction de la conversion en monomère.

Le tableau 2 ci-dessous présente les caractéristiques du polymère P(MAA-*co-*SS)-*b*-P(Sty) synthétisé.

**Tableau 2**

| Temps (h) | Conversion (%) | Mₙ, expérimental (g.mol⁻¹) | Mn théorique (g.mol⁻¹) | Iₚ |
|---|---|---|---|---|
| 5,5 | 38 | 58 350 | 65 750 | 1,44 |

La Mₙ expérimentale est déterminée par chromatographie d'exclusion stérique dans le DMF contenant 1g.L⁻¹ de LiBr, avec une calibration au polyméthacrylate de méthyle, après méthylation des unités acide méthacryliques en unités méthacrylate de méthyle.

### Exemple 2 comparatif: procédé d'obtention de micelles sphériques

L'exemple 2 illustre la synthèse de particules sous formes de micelles sphériques de copolymères à blocs poly(méthacrylate de sodium-*co*-styrène sulfonate de sodium)-*b-*poly(méthacrylate de méthyle-*co*-styrène) à partir du macroamorceur préparé et isolé dans l'exemple 1.

Dans un ballon monocol de 250 mL sont introduits 41,5 g d'eau permutée, 4,1 g de macroamorceur P(MAA-*co*-SS)-SG1 (8,14×10⁻³ mol.L⁻¹eau) préparé dans l'exemple 1, 37,8 g de soude 1M (1 équivalent par rapport aux unités acide méthacrylique) et 0,29 g de Na₂CO₃ (3,46×10⁻² mol.L⁻¹). Ce mélange est agité à température ambiante, pendant 15 minutes environ, jusqu'à dissolution complète du macroamorceur, qui est alors sous la forme poly(méthacrylate de sodium-*co*-styrène sulfonate de sodium). 18,2 g de méthacrylate de méthyle et 1,9 g de styrène sont ensuite ajoutés (taux de solide = 19,2%) et le mélange est dégazé par un bullage d'azote à température ambiante pendant 30 minutes.

Le milieu est introduit dans un réacteur chaud sous une pression de 3 bar en azote et une agitation à 250 rpm. Le temps t=0 est déclenché à 60°C et le réacteur est maintenu à 90°C tout le long de la polymérisation. Des prélèvements sont réalisés à intervalles réguliers afin de :
- déterminer la cinétique de polymérisation par gravimétrie (mesure d'extrait sec) ;
- suivre l'évolution des masses molaires moyennes en nombre (Mn) avec la conversion en monomères ;
- évaluer les caractéristiques colloïdales du latex (par Microcopie Electronique à Transmission (MET) et par diffusion de la lumière : diamètre moyen des particules, distribution en taille des particules (polydispersité)).

Le tableau 3 ci-dessous présente les caractéristiques des latex prélevés. Le latex obtenu en fin de polymérisation est transparent et est très peu visqueux.

L'aspect des particules est analysé par microscopie électronique à transmission. Ce microscope est de type JEOL 100 Cx II à 100 keV équipé avec une caméra haute résolution CCD camera Keen View de SIS. L'image obtenue est présentée dans la figure 1 annexée (67,2% en conversion massique avec un taux de 23,4% en poids en fraction hydrophile).

**Tableau 3**

| Temps (h) | Conversion (%) | M_{n,exp}^{a} g.mol⁻¹ | Mₙ, théo^{b} g.mol⁻¹ | Iₚ^{a} | pH | Dz^{c} (nm) | ∑^{d} |
|---|---|---|---|---|---|---|---|
| 0,25 | 17,6 | 19 700 | 12 700 | 1,27 | - | - | - |
| 0,5 | 26,9 | 23 150 | 15 570 | 1,37 | 7 | - | - |
| 0,75 | 37,6 | 31 700 | 18 900 | 1,2 | - | - | - |
| 1 | 56,2 | 35 960 | 24 700 | 1,23 | - | - | - |
| 2,9 | 67,2 | 40 500 | 28 000 | 1,29 | 6,85 | 36,8 | 0,16 |

(a) Déterminées par chromatographie d'exclusion stérique dans le DMF avec 1g.L⁻¹ de LiBr, avec calibration au polyméthacrylate de méthyle, après méthylation des unités acide méthacryliques en unités méthacrylate de méthyle ;
(b) Calculées à partir d'unités méthacrylate de méthyle ;
(c) Diamètre moyen en intensité des particules ;
(d) Polydispersité des latex ;

### Exemple 3 : procédé d'obtention de particules filamenteuses selon l'invention

L'exemple 3 illustre la synthèse de particules filamenteuses de copolymères blocs poly(méthacrylate de sodium-*co*-styrène sulfonate de sodium)-*b*-poly(méthacrylate de méthyle-*co*-styrène) à partir du macroamorceur préparé et purifié dans l'exemple 1.

Dans un ballon monocol de 250 mL sont introduits 55,7 g d'eau permutée, 2,29 g de macroamorceur P(MAA-*co*-SS)-SG₁ (4,54×10⁻³ mol.L⁻¹eau) préparé dans l'exemple 1, 23,7 g de soude 1M (1 équivalent par rapport aux unités acide méthacrylique) et 0,295 g de Na₂CO₃ (3,5 x 10⁻² mol.L⁻¹). Ce mélange est agité à température ambiante, pendant 15 minutes environ, jusqu'à dissolution complète du macroamorceur, qui est alors sous la forme poly(méthacrylate de sodium-*co*-styrène sulfonate de sodium). 18,2 g de méthacrylate de méthyle et 1,8 g de styrène sont ensuite ajoutés (taux de solide = 19,5%) et le mélange est dégazé par un bullage d'azote à température ambiante pendant 30 minutes.

Le milieu est alors introduit dans un réacteur Parr^{®}, série 5100, muni d'une cuve en verre simple enveloppe de 300 mL d'un diamètre intérieur de 63 mm et d'une hauteur utile de 102 mm. L'agitation est maintenue par un agitateur à entraînement magnétique munie d'une turbine à 250 rpm. La cuve du réacteur est préalablement chauffée.

Le milieu est introduit dans le réacteur chaud sous une pression de 3 bar en azote et le temps t=0 est déclenché à 60°C et est maintenu à 90°C tout le long de la polymérisation. Des prélèvements sont réalisés à intervalles réguliers afin de :
- déterminer la cinétique de polymérisation par gravimétrie (mesure d'extrait sec) ;
- suivre l'évolution des masses molaires moyennes en nombre (Mn) avec la conversion en monomères ;
- évaluer les caractéristiques colloïdales du latex (par MET).

Le tableau 4 ci-dessous présente les caractéristiques des latex prélevés.

**Tableau 4**

| Temps (h) | Conversion (%) | Mₙ,ₑₓₚ^{a} g.mol⁻¹ | Mₙ, théo^{b} g.mol⁻¹ | Iₚ^{a} | pH |
|---|---|---|---|---|---|
| 0,25 | 18 | 23 900 | 17 200 | 1,3 | 7,9 |
| 0,5 | 25,5 | 31 600 | 21 350 | 1,24 | - |
| 0,75 | 43,6 | 42 850 | 31 400 | 1,13 | 7,55 |
| 1 | 52 | 46 700 | 36 000 | 1,13 | - |
| 3,1 | 68 | 53 700 | 44 900 | 1,2 | 6,7 |

(a) Déterminées par chromatographie d'exclusion stérique dans le DMF avec 1g.L⁻¹ de LiBr, avec une calibration au polyméthacrylate de méthyle, après méthylation des unités acide méthacryliques en unités méthacrylate de méthyle ;
(b) Calculées à partir d'unités méthacrylate de méthyle.

Le latex obtenu en fin de polymérisation est blanc est très visqueux.

L'aspect des particules est analysé par microscopie électronique à transmission (MET). Ce microscope est de type JEOL 100 Cx II à 100 keV équipé avec une caméra haute résolution CCD camera Keen View de SIS. Les images obtenues sont présentées dans les figures 2,3 et 4 comme indiqué ci-dessous :
- la figure 2 correspond à 25,5% en conversion massique avec un taux de 31% en poids en fraction hydrophile ;
- la figure 3 correspond à 52% en conversion massique avec un taux de 18% en poids en fraction hydrophile ;
- la figure 4 correspond à 68% en conversion massique avec un taux de 14,4% en poids en fraction hydrophile.

### Exemple 4 comparatif: procédé d'obtention de particules filamenteuses de type « poches »

L'exemple 4 illustre la synthèse de nanoparticules chevelues sous formes de 'poches' de copolymères blocs poly(méthacrylate de sodium-*co*-styrène sulfonate de sodium)-*b-*poly(méthacrylate de méthyle-*co*-styrène) à partir du macroamorceur préparé et purifié dans l'exemple 1.

Dans un ballon monocol de 250 mL sont introduits 65,8 g d'eau permutée, 1,1765 g de macroamorceur P(MAA-*co*-SS)-SG1 (2,33×10⁻³ mol.L⁻¹ₑₐᵤ) préparé dans l'exemple 1, 13,7 g de soude 1M (1 éq. par rapport aux unités acide méthacrylique) et 0,299 g de Na₂CO₃ (3,54×10⁻² mol.L⁻¹). Ce mélange est agité à température ambiante, pendant 15 minutes environ, jusqu'à dissolution complète du macroamorceur, qui est alors sous la forme poly(méthacrylate de sodium-*co*-styrène sulfonate de sodium). 18,1 g de méthacrylate de méthyle et 1,9 g de styrène sont ensuite ajoutés (taux de solide = 19,75%) et le mélange est dégazé par un bullage d'azote à température ambiante pendant 30 minutes.

Le milieu est introduit dans un réacteur chaud (même configuration que dans l'exemple 2 sous une pression de 3 bar en azote et une agitation à 250 rpm. Le temps t=0 est déclenché à 60°C et le réacteur est maintenu à 90°C tout le long de la polymérisation. Des prélèvements sont réalisés à intervalles réguliers afin de :
- déterminer la cinétique de polymérisation par gravimétrie (mesure d'extrait sec) ;
- suivre l'évolution des masses molaires moyennes en nombre (Mn) avec la conversion en monomères ;
- évaluer les caractéristiques colloïdales du latex (par Microcopie Electronique à Transmission).

Le tableau 5 ci-dessous présente les caractéristiques des latex prélevés.

**Tableau 5**

| Temps (h) | Conversion (%) | M_{n,exp}^{a} g.mol⁻¹ | Mₙ, théo^{b} g.mol⁻¹ | Iₚ^{a} | pH |
|---|---|---|---|---|---|
| 0,25 | 10,2 | 29 350 | 18200 | 1,23 | 6,7 |
| 0,56 | 21,4 | 60 000 | 30 300 | 1,24 | - |
| 1 | 41,3 | 78 860 | 51 800 | 1,2 | - |
| 3,1 | 53,4 | 80 700 | 64 850 | 1,2 | 6,6 |

(a) Déterminées par chromatographie d'exclusion stérique dans le DMF avec 1g.L⁻¹ de LiBr, avec calibration au polyméthacrylate de méthyle, après méthylation des unités acide méthacryliques en unités méthacrylate de méthyle ;
(b) Calculées à partir d'unités méthacrylate de méthyle ;

Le latex obtenu en fin de polymérisation est blanc et est légèrement visqueux.

L'aspect des particules est analysé par MET. Ce microscope est de type JEOL 100 Cx II à 100 keV équipé avec une caméra haute résolution CCD camera Keen View de SIS. Les images obtenues sont présentées dans la figure 6 et correspondent à 53,4% conversion massique avec un taux de 10% en poids en fraction hydrophile.

### Exemple 5 selon l'invention : procédé d'obtention de particules filamenteuses réticulées

L'exemple 5 illustre la synthèse de particules filamenteuses contenant du réticulant, de copolymères blocs poly(méthacrylate de sodium-*co*-styrène sulfonate de sodium)-*b-*poly(méthacrylate de méthyle-*co*-styrène-co-divinylbenzène) à partir du macroamorceur préparé dans l'exemple 1.

Dans un ballon monocol de 250 mL sont introduits 57 g d'eau permutée, 2,63 g de macroamorceur P(MAA-*co*-SS)-SG1 (5.22×10⁻³ mol.L⁻¹eau) préparé dans l'exemple 1, 24,2 g de soude 1M (1 équivalent par rapport aux unités acide méthacrylique) et 0,296 g de Na₂CO₃ (3,45 ×10⁻² mol.L⁻¹). Ce mélange est agité à température ambiante, pendant 15 minutes environ, jusqu'à dissolution complète du macroamorceur, qui est alors sous la forme poly(méthacrylate de sodium-*co*-styrène sulfonate de sodium). 18,1 g de méthacrylate de méthyle et 0,784 g de styrène (f_{0,sty}= 0,04 mol ; *f*_{0*,sty*} = *n_{Sty}*/(*n_{sty}* + *n_{MMA}*)) sont ensuite ajoutés et le mélange est dégazé par un bullage d'azote à température ambiante pendant 30 minutes.

Le milieu est introduit dans un réacteur chaud (même configuration que dans l'exemple 2 sous une pression de 3 bar en azote et une agitation à 250 rpm. Le temps t=0 est déclenché à 60°C et le réacteur et est maintenu à 90°C tout le long de la polymérisation.

Au bout de 50 minutes, 0,982 g de divinylbenzène (f_{0,DVP} = 0,04 mol) (*f_{0,DVP}* = *n_{DVP}*/*(n_{DVP}* + *n_{MMA}*) (taux de solide = 19%) sont introduits dans le milieu pour réticuler les fibres après leur formation.

Des prélèvements sont réalisés à intervalles réguliers afin de :
- déterminer la cinétique de polymérisation par gravimétrie (mesure d'extrait sec) ;
- suivre l'évolution des masses molaires moyennes en nombre (Mn) avec la conversion en monomères.

Le tableau 6 ci-dessous présente les caractéristiques des latex prélevés.

**Tableau 6**

| Temps (h) | Conversion (%) | Mₙ, ₑₓₚ^{a} g.mol⁻¹ | Mₙ, théo^{b} g.mol⁻¹ | Iₚ^{a} | pH |
|---|---|---|---|---|---|
| 0,25 | 19 | - | 15 700 | - | 7,8 |
| 0,5 | 26 | - | 18 800 | - | - |
| 0,75 | 45 | - | 27 300 | - | - |
| 1 | 52 | - | 31 600 | - | - |
| 3 | 65 | - | 37700 | - | - |

(a) Déterminées par chromatographie d'exclusion stérique dans le DMF avec 1g.L⁻¹ de LiBr, avec calibration au polyméthacrylate de méthyle, après méthylation des unités acide méthacryliques en unités méthacrylate de méthyle ;
(b) Calculées à partir d'unités méthacrylate de méthyle.

Le latex obtenu en fin de polymérisation est sous forme de gel. Le réticulant est ajouté en cours de polymérisation pour permettre l'autoassemblage des particules avant la réticulation les micelles sous formes de micelles allongées.

Les images obtenues sont présentées dans la figure 7 qui correspond à 68% en conversion massique avec un taux de 14,4% en poids en fraction hydrophile.

### Exemple 6 - Etude du comportement des particules filamenteuses selon l'invention en milieu dispersé, en variant le pH et la salinité

Des particules filamenteuses préparées dans une solution aqueuse à 0,2% en poids sont dialysées pendant 4 jours contre de l'eau ultra pure. La figure 7 correspond à l'image MET de particules filamenteuses dialysées.

Des particules filamenteuses préparées dans une solution aqueuse à 0,2% en poids sont centrifugées pendant 1 heure à 2 100 rpm et à 10°C. Le sédiment (ou culot de centrifugation) obtenu est dilué dans une solution aqueuse. La figure 8 correspond à l'image MET de ces particules après centrifugation.

La figure 9 correspond à l'image TEM de particules filamenteuses diluées dans une solution à pH 5.

La figure 10 correspond à l'image TEM de particules filamenteuses diluées dans une solution aqueuse contenant 35gr de sel (NaCl) pour 1kg d'eau.

Ces résultats montent que les particules filamenteuses selon l'invention voient leur forme et structure se maintenir en milieu dispersé, indépendamment de leur concentration dans le milieu, de la dialyse des particules (figure 7), de la centrifugation du milieu (figure 8) et/ou des variations de pH (figure 9) ou de salinité de celui-ci (figure 10).

### Exemple 7 : étude des propriétés rhéologiques des particules filamenteuses selon l'invention

Les propriétés rhéologiques sont mesurées à l'aide d'un rhéomètre à contrainte imposée type AtonPaar MCR301. Les mesures d'écoulement (viscosité en fonction de taux de cisaillement) sont réalisées à température ambiante (25°C) avec une géométrie couette ou plan-plan (en fonction de la gamme de viscosité). L'exemple décrit les propriétés rhéologiques et rhéofluidifiantes données par les micelles fibrillaires allongées en comparaison à des structures de micelles classiques tel que des sphères ou des vésicules (ou poches). Les détails de ces structures, via des photos de microscopie par transmission sont exposés dans le tableau suivant :
Les valeurs de viscosité sont présentées dans le tableau 7 ci-dessous. Le comportement est newtonien lorsque la viscosité est indépendante de taux de cisaillement. Dans le cas du comportement rhéofluidifiant, la viscosité décroît avec l'augmentation du taux de cisaillement.

Ces résultats montrent la différence de comportement rhéologique en fonction de la structure des particules polymériques : les micelles sphériques (exemple 2) et les « poches » (exemple 4) sont dispersées sans percolation dans le milieu, présentent un comportement newtonien et une faible influence sur la viscosité. Les particules polymériques filamenteuses selon l'invention (latex final de l'exemple 3), présentent un comportement de solide viscoélastique (à 19,6%), là où les autres morphologies donnent un comportement de liquide. Ce comportement est dû à une « percolation » de ces longues micelles et engendre, même à des faibles concentrations (3 et 2g.L⁻¹), un comportement de solution rhéofluidifiante. Ce comportement s'est maintenu même après re-dilution des particules centrifugées.

**Tableau 7**

| Echantillon | Comportement rhéologique | η à 10⁻¹ s⁻¹ mPa.s | η à 10s⁻¹ mPa.s |
|---|---|---|---|
| Latex final brut de l'exemple 3 (fibres) Taux de solide = 19.6% | rhéofluidifiant | 870000 | 23000 |
| Latex final de l'exemple 3 dilué dans eau distillée concentration = 3g/L⁻¹ | rhéofluidifiant | 160 | 4 |
| Latex final de l'exemple 3 dilué dans eau distillée concentration = 2g/L⁻¹ | rhéofluidifiant | 50 | 2 |
| Latex final brut de l'exemple 2 (sphères) Taux de solide = 19.6% | Newtonien | 8 | 7 |
| Latex final de l'exemple 2 dilué dans eau distillée concentration = 2g/L⁻¹ | Newtonien | 4 | 2 |
| Latex final brut de l'exemple 4 (poches) Taux de solide = 19.6% | Newtonien | 2.3 | 2 |
| Latex final de l'exemple 4 dilué dans eau distillée concentration = 2g/L⁻¹ | Newtonien | 1,5 | 1,5 |
| Eau distillée | Newtonien | 1 | 1 |

### Abréviations :

PRC - polymérisation radicalaire contrôlée
P4VP - poly(4-vinylpyridine)
PNaA - poly(acrylate de sodium)
SG1 -N-tertiobutyl-N-[1-diethylphosphono-(2,2-diméthylpropyl)]
S ou Sty - styrene
SS - styrène sulfonate de sodium
AA - acide acrylique
PEGA - méthyl éther acrylate de poly(ethylene glycol)
TEM - microscopie électronique en transmission
RAFT - polymérisation par addition fragmentation (« Reversible Addition Fragmentation chain Transfer »)
MAA - acide méthacrylique
DMSO - dimethylsulfoxyde
DMF - diméthylformamide
Rpm - rotations par minute
f_{0,sty} - fraction molaire initiale de styrène dans le mélange des monomères
fo,ss - fraction molaire initiale de styrène dans le mélange des monomères
f_{0,DVP} - fraction molaire initiale de divinylbenzène dans le mélange des monomères BlocBuilder^{®}-MA - (*N*-(2-methylpropyl)-*N*-(1-diethylphosphono-2,2-dimethylpropyl)-*O*-(2-carboxylprop-2-yl) hydroxylamine

## Revendications

1. Particules polymériques filamenteuses ayant un ratio longueur/diamètre supérieur à 100 constituées de copolymères à blocs synthétisés par polymérisation radicalaire contrôlée en émulsion à partir d'au moins un monomère hydrophobe en présence d'un macroamorceur vivant dérivé d'un nitroxyde, **caractérisées en ce que** :
- lesdites particules filamenteuses sont obtenues en milieu aqueux lors de la synthèse desdits copolymères à blocs effectuée en chauffant le milieu de réaction à une température de 60 à 120°C,
- ledit macroamorceur est hydrosoluble,
- le pourcentage de la masse molaire du macroamorceur hydrosoluble dans le copolymère à blocs final est compris entre 10 et 50%, et **en ce que**
- le taux de conversion du monomère hydrophobe est d'au moins 50%.

2. Particules selon la revendication 1 ayant une longueur supérieure à 500 nm, de préférence supérieure à 1 micron, avantageusement supérieure à 5 microns.

3. Particules selon l'une des revendications 1 à 2 dans lesquelles le monomère hydrophobe est choisi parmi les monomères vinylaromatiques tels que le styrène ou les styrènes substitués, les acrylates d'alkyle, de cycloalkyle ou d'aryle tels que l'acrylate de méthyle, d'éthyle, de butyle, d'éthylhexyle ou de phényle, les méthacrylates d'alkyle, de cycloalkyle, d'alkényle ou d'aryle tels que le méthacrylate de méthyle, de butyle, de lauryle, de cyclohexyle, d'allyle ou de phényle et la vinylpyridine.

4. Particules selon l'une des revendications 1 à 3 dans lesquelles le taux massique de la partie hydrophile composant le copolymère à bloc final est inférieur à 25%.

5. Particules selon l'une des revendications 1 à 4 dans lesquelles le pourcentage de la masse molaire du macroamorceur hydrosoluble dans le copolymère à blocs final est compris entre 10 et 30%.

6. Particules selon l'une des revendications 1 à 5 comprenant en outre un comonomère réticulant.

7. Particules selon la revendication 6 dans lesquelles le comonomère réticulant est choisi parmi les divinylbenzènes, les trivinylbenzènes, les (méth)acrylates allyliques, les (méth)acrylates de diallylmaléatepolyol et les di(méth)acrylates d'alkylèneglycol qui ont de 2 à 10 atomes de carbone dans la chaîne carbonée.

8. Particules selon la revendication 1 **caractérisées en ce que** leur synthèse est effectuée par polymérisation radicalaire par transfert réversible par addition fragmentation (RAFT) dans l'eau à partir d'au moins un monomère hydrophobe en présence d'un agent RAFT macromoléculaire (ou macroagent RAFT) hydrophile.

9. Procédé pour modifier la rhéologie d'une solution aqueuse ou organique au moyen des particules polymériques filamenteuses définies selon l'une des revendications 1 à 8, ledit procédé comprenant une étape d'ajout de ces particules filamenteuses à une solution aqueuse ou organique à un taux massique de minimum 100 ppm, de préférence de 500 à 10000 ppm.

10. Procédé d'extraction renforcée d'hydrocarbures au moyen d'un additif polymérique dans lequel on mélange ledit additif avec de l'eau ou de la saumure dans une proportion d'au moins 500 ppm d'additif puis on injecte ce mélange sous pression dans la roche, **caractérisé en ce que** ledit additif consiste en les particules polymériques filamenteuses définies dans une des revendications 1 à 8.

11. Composition pour modifier la rhéologie d'une solution aqueuse ou organique, ladite composition comprenant les particules polymériques filamenteuses définies dans une des revendications 1 à 8.

12. Utilisation de la composition selon la revendication 11 dans la préparation de peintures.

13. Utilisation de la composition selon la revendication 11 dans la préparation de produits cosmétiques.

## Patentansprüche

1. Filamentöse Polymerpartikel, die ein Länge/Durchmesser-Verhältnis von mehr als 100 aufweisen und aus Blockcopolymeren bestehen, die durch kontrollierte radikalische Emulsionspolymerisation ausgehend von zumindest einem hydrophoben Monomer in Gegenwart eines lebenden, von einem Nitroxid abgeleiteten Makroinitiators synthetisiert werden, **dadurch gekennzeichnet, dass**:
- die filamentösen Partikel in einem wässrigen Medium während der Synthese der Blockcopolymere erhalten werden, die unter Erwärmen des Reaktionsmediums auf eine Temperatur von 60 bis 120 °C durchgeführt wird,
- der Makroinitiator wasserlöslich ist,
- der prozentuale Anteil der Molmasse des wasserlöslichen Makroinitiators in dem fertigen Blockcopolymer zwischen 10 und 50 % liegt, und dadurch, dass:
- der Umwandlungsgrad des hydrophoben Monomers mindestens 50 % beträgt.

2. Partikel nach Anspruch 1, die eine Länge von mehr als 500 nm, vorzugsweise über 1 Mikrometer und vorteilhaft über 5 Mikrometern aufweisen.

3. Partikel nach einem der Ansprüche 1 oder 2, wobei das hydrophobe Monomer unter vinylaromatischen Monomeren, wie Styrol oder substituierten Styrolen, Alkylacrylaten, Cycloalkylacrylaten oder Arylacrylaten, wie Methylacrylat, Ethylacrylat, Butylacrylat, Ethylhexylacrylat oder Phenylacrylat, Alkylmethacrylaten, Cycloalkylmethacrylaten, Alkenylmethacrylaten oder Arylmethacrylaten, wie Methylmethacrylat, Butylmethacrylat, Laurylmethacrylat, Cyclohexylmethacrylat, Allylmethacrylat oder Phenylmethacrylat, und Vinylpyridin ausgewählt ist.

4. Partikel nach einem der Ansprüche 1 bis 3, wobei der Gewichtsanteil der hydrophilen Einheit, aus der das fertige Blockcopolymer gebildet ist, unter 25 % liegt.

5. Partikel nach einem der Ansprüche 1 bis 4, wobei der prozentuale Anteil der Molmasse des wasserlöslichen Makroinitiators in dem fertigen Blockcopolymer zwischen 10 und 30 % liegt.

6. Partikel nach einem der Ansprüche 1 bis 5, die ferner ein vernetzendes Comonomer enthalten.

7. Partikel nach Anspruch 6, wobei das vernetzende Comonomer unter Divinylbenzolen, Trivinylbenzolen, Allyl(meth)acrylaten, Diallylmaleatpolyol(meth)acrylaten und Alkylenglycol(meth)-acrylaten, die 2 bis 10 Kohlenstoffatome in der Kohlenstoffkette aufweisen, ausgewählt ist.

8. Partikel nach Anspruch 1, **dadurch gekennzeichnet, dass** die Synthese durch radikalische RAFT (Reversible Addition-Fragmentation Chain Transfer)-Polymerisation in Wasser ausgehend von zumindest einem hydrophoben Monomer in Gegenwart eines hydrophilen makromolekularen RAFT-Agens (oder RAFT-Makroagens) erfolgt.

9. Verfahren zur Modifizierung der Rheologie einer wässrigen oder organischen Lösung mit Hilfe von filamentösen Polymerpartikeln nach einem der Ansprüche 1 bis 8, wobei das Verfahren einen Schritt der Zugabe der filamentösen Partikel in eine wässrige oder organische Lösung in einem Masseanteil von wenigstens 100 ppm und vorzugsweise 500 bis 10000 ppm umfasst.

10. Verfahren zur verbesserten Kohlenwasserstoff-Extraktion mit Hilfe eines polymeren Additivs, wobei das Additiv in einem Mengenanteil von mindestens 500 ppm Additiv mit Wasser oder Salzlösung gemischt und dieses Gemisch dann unter Druck in Gestein injiziert wird, **dadurch gekennzeichnet, dass** das Additiv aus den in einem der Ansprüche 1 bis 9 definierten filamentösen Polymerpartikeln besteht.

11. Zusammensetzung zur Modifizierung der Rheologie einer wässrigen oder organischen Lösung, wobei die Zusammensetzung die in einem der Ansprüche 1 bis 9 definierten filamentösen Polymerpartikel enthält.

12. Verwendung der Zusammensetzung nach Anspruch 11 zur Herstellung von Farben und Lacken.

13. Verwendung der Zusammensetzung nach Anspruch 11 zur Herstellung von kosmetischen Produkten.

## Claims

1. Filamentous polymer particles having a length/diameter ratio of more than 100 and composed of block copolymers synthesized by controlled radical emulsion polymerization from at least one hydrophobic monomer in the presence of a living macroinitiator derived from a nitroxide, wherein:
- said filamentous particles are obtained in aqueous medium during the synthesis of said block copolymers, formed by heating the reaction medium at a temperature of 60 to 120°C,
- said macroinitiator is water-soluble,
- the percentage of the molar mass of the water-soluble macroinitiator in the final block copolymer is between 10% and 50%, and in that
- the degree of conversion of the hydrophobic monomer is at least 50%.

2. The particles as claimed in claim 1, having a length of more than 500 nm, preferably more than 1 micron, advantageously more than 5 microns.

3. The particles as claimed in any of claims 1 or 2, wherein the hydrophobic monomer is selected from vinylaromatic monomers such as styrene or substituted styrenes, alkyl, cycloalkyl, or aryl acrylates such as methyl, ethyl, butyl, ethylhexyl, or phenyl acrylate, alkyl cycloalkyl, alkenyl, or aryl methacrylates such as methyl, butyl, lauryl, cyclohexyl, allyl, or phenyl methacrylate, and vinylpyridine.

4. The particles as claimed in any of claims 1 to 3, wherein the percentage of the molar mass of the water-soluble macroinitiator in the final block copolymer is between 10% and 30%.

5. The particles as claimed in any of claims 1 to 4, wherein the mass fraction of the hydrophilic moiety constituting the final block copolymer is less than 25%.

6. The particles as claimed in any of claims 1 to 5, further comprising a crosslinking comonomer.

7. The particles as claimed in claim 6, wherein the crosslinking comonomer is selected from divinylbenzenes, trivinylbenzenes, allyl (meth)acrylates, diallyl maleate polyol (meth)acrylates, and alkylene glycol di(meth)acrylates which have from 2 to 10 carbon atoms in the carbon chain.

8. The particles as claimed in claim 1, **characterized in that** they are synthesized by radical polymerization by reversible addition fragmentation transfer (RAFT) in water from at least one hydrophobic monomer in the presence of a macromolecular RAFT agent (or RAFT macroagent) which is hydrophilic.

9. A method for modifying the rheology of an aqueous or organic solution by means of the filamentous polymer particles defined in any of claims 1 to 8, said method comprising a step of adding these filamentous particles to an aqueous or organic solution in a mass fraction of not less than 100 ppm, preferably of 500 to 10 000 ppm.

10. A method of enhanced hydrocarbon extraction by means of a polymeric additive, wherein said additive is mixed with water or brine in a proportion of at least 500 ppm of additive and then this mixture is injected under pressure into the rock, **characterized in that** said additive comprises the filamentous polymer particles defined in any of claims 1 to 8.

11. A composition for modifying the rheology of an aqueous or organic solution, said composition comprising the filamentous polymer particles defined in any of claims 1 to 8.

12. The use of the composition as claimed in claim 11 in the preparation of paints.

13. The use of the composition as claimed in claim 11 in the preparation of cosmetic products.
